# EUROPEAN PATENT SPECIFICATION

(11) **EP 1 748 789 B1**
(45) Date of publication and mention of the grant of the patent: **29.12.2010**
(21) Application number: 04803279.1
(22) Date of filing: 05.11.2004
(51) Int. Cl.: A61K 38/20, A61P 31/00, A61P 31/18

(54) **IL-22 for preventing infectious diseases**
IL-22 zur Verhinderung von Infektionskrankheiten
IL-22 pour la prévention des maladies infectieuses

(30) Priority: 05.11.2003 US 517104 P; 21.06.2004 US 580720 P
(43) Date of publication of application: 07.02.2007
(73) Proprietor: Institut de Recherche pour le Développement ( IRD), 75480 Paris Cedex 10 (FR); Immunoclin Ltd, London N12 8NP (GB)
(72) Inventor: VEAS, Francisco, F-34130 Maugio (FR); MISSE, Dorothée, F-34070 Montpellier (FR); CLERICI, Mario, I-20129 Milano (IT); TRABATONI, Daria, I-20033 Desio (IT)
(74) Representative: Peaucelle, Chantal
(86) International application number: PCT/EP2004/013393
(87) International publication number: WO 2005/044292

(56) References cited:
- WO-A-02/29098
- WO-A-99/61617
- WO-A-02/060468
- US-A1- 2001 023 070
- MASIHI K N: "Progress on novel immunomodulatory agents for HIV-1 infection and other infectious diseases" EXPERT OPINION ON THERAPEUTIC PATENTS 01 JUN 2003 UNITED KINGDOM, vol. 13, no. 6, 1 June 2003 (2003-06-01), pages 867-882, XP002328920 ISSN: 1354-3776
- LANE BRIAN R ET AL: "Human immunodeficiency virus type 1 (HIV-1)-induced GRO-alpha production stimulates HIV-1 replication in macrophages and T lymphocytes" JOURNAL OF VIROLOGY, vol. 75, no. 13, July 2001 (2001-07), pages 5812-5822, XP002328921 ISSN: 0022-538X
- LE Y ET AL: "Receptors for chemotactic formyl peptides as pharmacological targets" INTERNATIONAL IMMUNOPHARMACOLOGY, ELSEVIER, AMSTERDAM, NL, vol. 2, no. 1, January 2002 (2002-01), pages 1-13, XP002249587 ISSN: 1567-5769
- RICHARDSON RICARDO M ET AL: "Interleukin-8-mediated heterologous receptor internalization provides resistance to HIV-1 infectivity. Role of signal strength and receptor desensitization." JOURNAL OF BIOLOGICAL CHEMISTRY, vol. 278, no. 18, 2 May 2003 (2003-05-02), pages 15867-15873, XP002328922 ISSN: 0021-9258 cited in the application
- LI B-Q ET AL: "The synthetic peptide WKYMVm attenuates the function of the chemokine receptor CCR5 and CXCR4 through activation of formyl peptide receptor-like 1" BLOOD, W.B.SAUNDERS COMPANY, ORLANDO, FL, US, vol. 97, no. 10, 25 May 2001 (2001-05-25), pages 2941-2947, XP002972742 ISSN: 0006-4971
- BRIGGS S D ET AL: "HIV-1 Nef promotes survival of myeloid cells by a Stat3-dependent pathway." THE JOURNAL OF BIOLOGICAL CHEMISTRY. 6 JUL 2001, vol. 276, no. 27, 6 July 2001 (2001-07-06), pages 25605-25611, XP002328923 ISSN: 0021-9258
- WEI C-C ET AL: "CLONING AND CHARACTERIZATION OF MOUSE IL-22 BINDING PROTEIN" GENES AND IMMUNITY, NATURE PUBLISHING GROUP, GB, vol. 4, no. 3, April 2003 (2003-04), pages 204-211, XP009033667 ISSN: 1466-4879
- DATABASE BIOSIS [Online] BIOSCIENCES INFORMATION SERVICE, PHILADELPHIA, PA, US; March 2003 (2003-03), KOHLER JAMES J ET AL: "Human immunodeficiency virus type 1 (HIV-1) induces activation of multiple STATs in CD4+ cells of lymphocyte or monocyte/macrophage lineages." XP002328990 Database accession no. PREV200300427253 & JOURNAL OF LEUKOCYTE BIOLOGY, vol. 73, no. 3, March 2003 (2003-03), pages 407-416, ISSN: 0741-5400
- MISSE D ET AL: "IL-22 participates in an innate anti-HIV-1 host-resistance network through acute-phase protein induction" , vol. 178, 2007, pages 407-415,
- THULASIRAMAN V ET AL: "Detection and identification of virulence factors in Yersinia pestis using SELDI ProteinChip system." BIOTECHNIQUES, vol. 30, no. 2, October 2001 (2001-10), pages 428-432,

## Description

The invention concerns biomarkers of resistance to infections in humans and biological applications thereof, particularly in diagnostics, prophylaxis and therapeutics.

It relates to biomarkers of resistance to infections due to pathogens in general, particularly infections due to virus and retrovirus, and more particularly to HIV-infections.

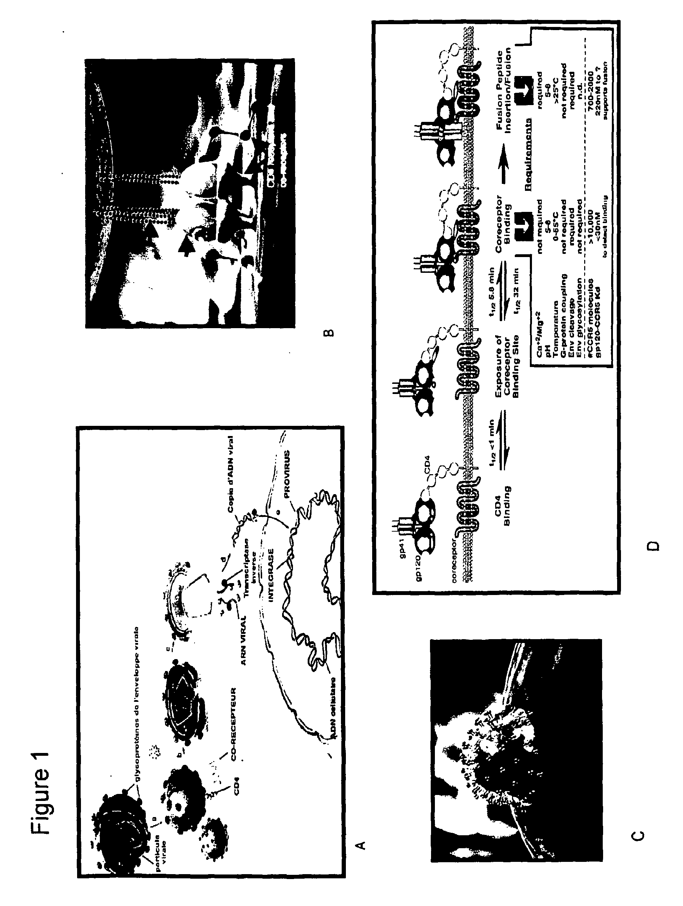

Several viral diseases emerged at the end of the twentieth century, particularly the Acquired Immunodeficiency Syndrome (AIDS) caused by the human immunodeficiency virus (HIV). More than two decades since its discovery, human immunodeficiency virus (HIV) epidemic is still a major burden for health, social and economical reasons on all over the world. During 2002, about 3.1 millions of deaths were listed, while about 5 millions of new infection cases were registered. Over 40 million people are infected worldwide and there is an urgent need to find agents to prevent the spread of this virus as well as to improve on the current treatment regimen. To date, both host genetic repertoire, innate and acquired immune responses, viral mutation or attenuation have been invoked to explain the higher or lower individual susceptibility to the infection. A great deal of progress has been made in understanding the mechanism of human immunodeficiency virus entry into target cells. Landmark discoveries such as the identification of viral coreceptors and the structure of the viral envelope protein (Env) bound to its receptor provided important insight into how Env mediates fusion of the viral and cellular membranes as described in **Fig. 1****.**

The existence of some people somewhat "immune" from infection, despite dealing with repeated HIV exposure, as well as the extremely slow disease progression in some HIV infected individuals, offers valuable clues to elucidate mechanisms underlying natural HIV resistance. Strikingly, both such cohorts, the so-called Exposed Seronegative, Exposed Uninfected (ESN, EU) and the Slow Progressors, Long Term Progressors (SP, LTNP) individuals have common immune responses, e.g. the generation of neutralising antibodies directed against common targets, which can play a protective role in virus entry and/or spread.

In 1989 a paper by Ranki described a curious phenomenon: HIV-specitic T-cell response to HIV, native gp 120 and recombinant envelope and core proteins could be detected in antibody- and antigen-negative sexual partners of known HIV-positive men. Two other reports confirmed that initial observation, and the authors raised the possibility that exposure to HIV that did not result in seroconversion and infection would be associated with the exclusive priming of T helper lymphocytes [2]. Analyses performed in different cohorts of individuals at high risk of HIV infection, and including health care workers parenterally exposed to HIV and healthy newborns of HIV-infected mothers, revealed that HIV-specific T helper cells, but not antibodies, were present in all these subjects. These observations led to the hypothesis that viral exposure resulting in the exclusive priming of HIV-specific T cells could be associated with protection against actual HIV infection. This hypothesis was greatly strengthened by three commercial sex workers in Narobi (the Pumwaani cohort), clearly demonstrated that whereas the majority of women who started to prostitute themselves became HIV infected within a year, a sizable minority, subsequently estimated to be around 15% of the individuals tested, was clearly resistant to infection. 2) Sarah Rowland-Jones showed the presence of HIV-specific CTL in healthy newborns of HIV infected mothers. The detection of HIV-specific, IFNã- secreting CD8 T lymphocytes in these newborns was a turning point in the realization that HIV exposure not associated with seroconversion is associated with an actual abortive infection and that live, replicating virus is indeed responsible for the stimulation of specific immunity. In fact, only actual infection with the virus would result in presentation of viral antigens in association with HLA class I molecules, and elicitation of a CD8-mediated immune response. (much later, the protective role of cell mediated immunity in this setting was further reinforced by the observation that late seroconversion occurring in Kenyan HIV-resistant sex workers who interrupt commercial sex work for a period of time is related to the waning of HIV-specific CD8+ responses due to reduced antigenic exposure) . 3) Experiments in which macaques exposed in vivo to subinfectious doses of SIV, and in whom SIV-specific T helper cells were detected, demonstrated protection against subsequent challenges with infectious doses of the same virus (these result were not unequivocally confirmed by other investigators).

The field of investigation of immune correlates of protection against HIV infection was born. Subsequent, pivotal reports showed that in HIV-exposed but uninfected individuals: 1) a particular genetic background, epitomized by the .32 deletion in the CCR5 receptor, could be present; 2) the production of soluble factors, including cell antiviral factors (CAF) , beta chemokines, and alpha defensins, is increased ; 3) secretory HIV-specific IgA as well as T helper and CTL can be detected in cervico-vaginal fluids and ejaculates [19], and 4) NK cell activity is particularly potent [2]. Thus, 15 years after the first description of the detection of HIV-specitic T helper cells in seronegative individuals, possible resistance to HIV infection can be summarized as being correlated with the elicitation of systemic and mucosal cell mediated immunity, and mucosally-confined IgA, possibly within favourable genetic and natural immunity settings.

**Table 1. Mechanisms suggested to be associated with resistance to HIV infection.**

| Acquired mechanisms | Genetic mechanisms | Innate Immunity |
|---|---|---|
| H I V-specific T helper cells | Deletion in the HIV-1 | Elevated NK activity |
| | Coreceptors | |
| HIV-specific CTL | Particular HLA alleles | Elevated production of β chemokines |
| Mucosal HIV-specific IgA | | Elevated production of CAF |
| Anti CD4 antibodies | | Elevated concentration of α defensins |
| Anti CCR5 antibodies | | |

The comprehension of mechanisms of natural resistance to HIV infection may have implications for the identification of anti-viral novel strategies and in particular for the development of innovative diagnostics, therapeutics and vaccine design.

The inventors have compared studies on protein profiles (proteom) and genome expression (transcriptome) from HIV exposed uninfected individuals (EU), HIV exposed and infeceted individuals (HIV+) and healthy donnors (HC) to identify biomarkers from EU that could explain resistance mechanisms to the HIV infection.

They have identified a key cytokine which appears to be responsible for the induction of proteins involved in resistance to viruses.

They have also found that another cytokine shows a polymorphism among the studied cohorts exhibiting a particular pattern in EU. Then other isoforms also appear to be involved in HIV resistance processes by their effect on FPR or FPRLI receptors and the subsequent phosphorylation of CCR5 or CXCR4 HIV-co-receptors. Then the combination of these cytokines was considered as element participating indirectly to the viral infection blockade. Individually and in combination, they also appear to participate in the HIV resistance *mechanisms.*

The invention relates to IL-22 for use in preventing infections diseases by initiating innate immune response.

Proteins of an innate immune response including II-22 the Jack/STAT pathway, SOCS3, beta defensines (2 and 3) and the acute phase apolipoprotein serum amyloid Ac or A-SAA or a fragment thereof, IL-8 cytokine and isoforms thereof, are of great value in biological applications in view of their properties as biomarkers of resistance to HIV infections. Particularly, they are of great interest in diagnostics, therapeutics and prophylaxis.

In the above mentioned use an effective amount of IL-22 is in association with a pharmaceutically acceptable carrier.

The pharmaceutical compositions of the invention are advantageously prepared for administration by the oral , intramuscular, intravenous or mucosal route.

For oral administration, they are presented in the form of tablets, pills, capsules, drops, patch or spray.

For administration by injection, the pharmaceutical compositions are under the form of solution for injection by the intravenous, subcutaneous or intramuscular route produced from sterile or sterilisable solution, or suspension or emulsion.

For administration by mucosal route, the pharmaceutical compositions are under the form of gels.

The administration doses will easily be adjusted by the one skilled in the art depending on the patient's condition.

According to still another aspect, the invention relates to a method for favouring the innate host resistance to viral infections, comprising using IL-22 as starter cytokine helping the innate immune response to infections.

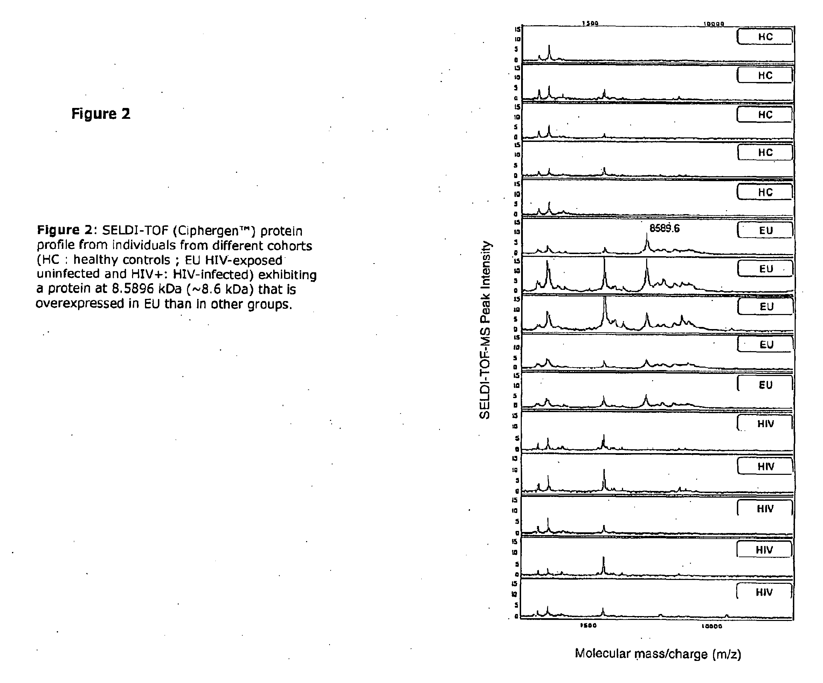

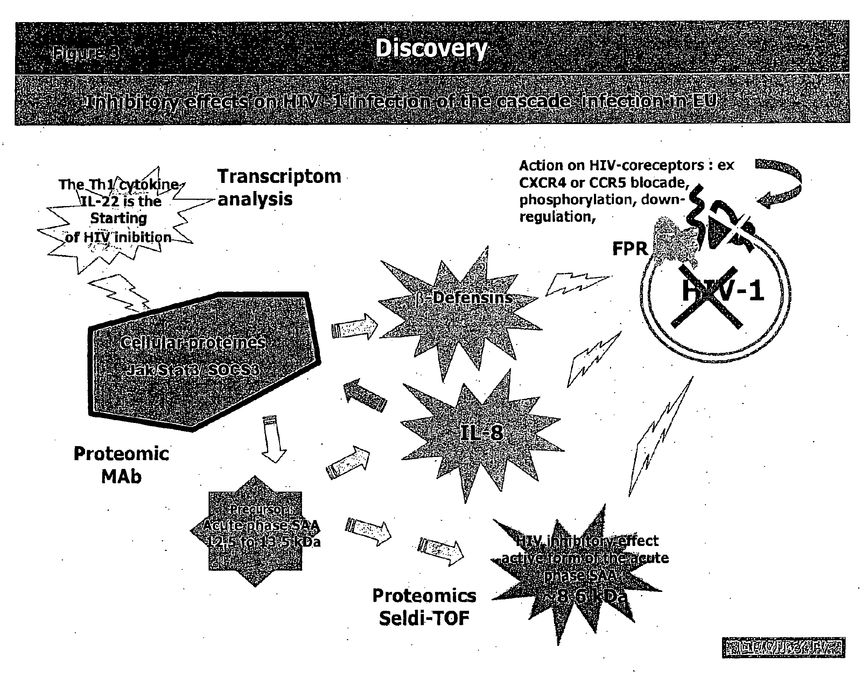

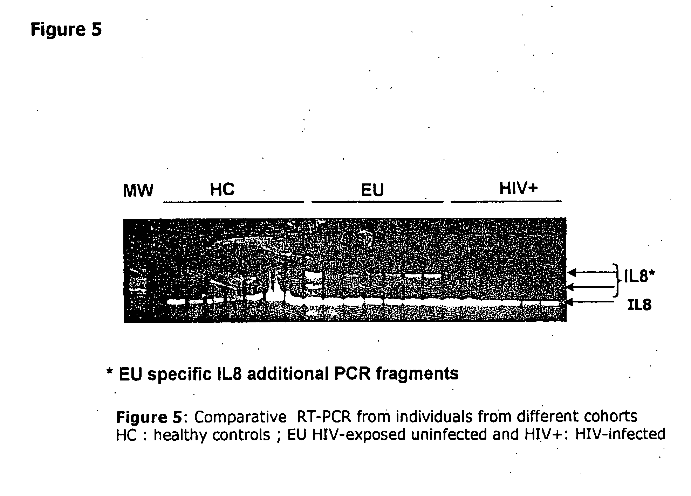

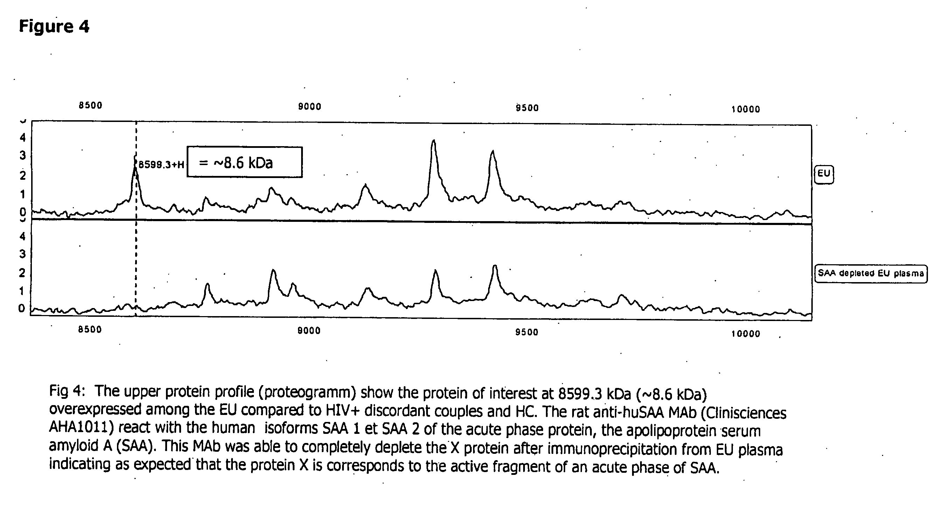

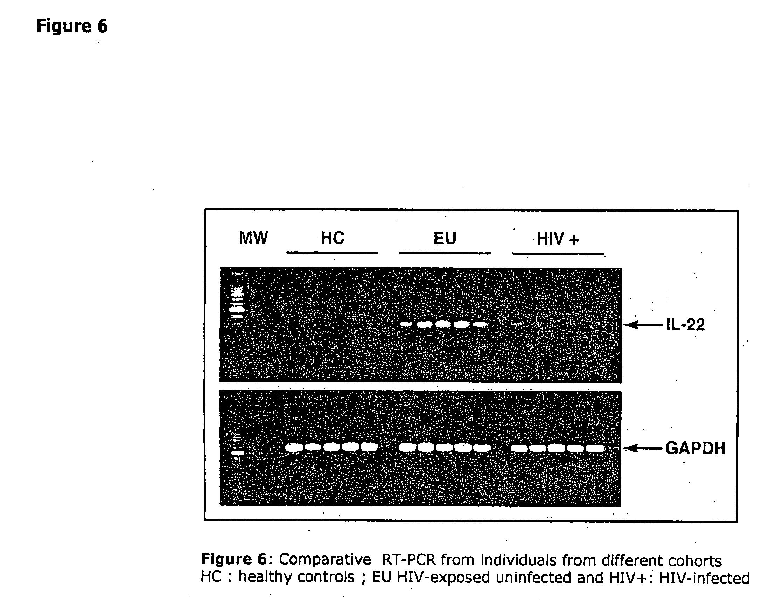

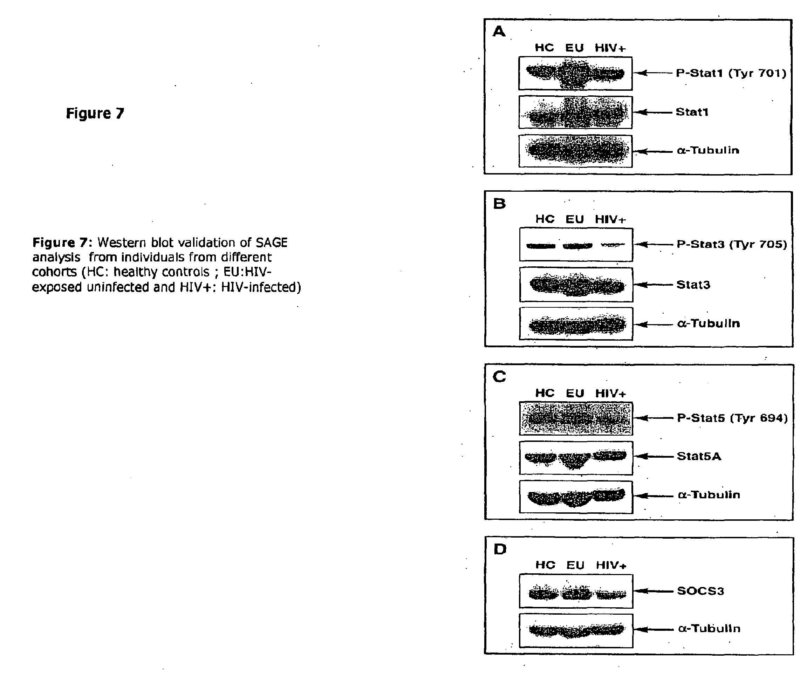

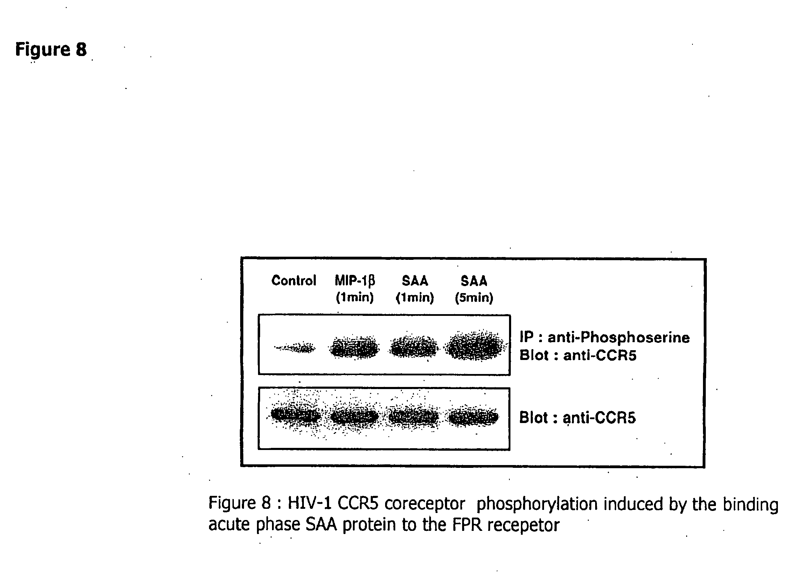

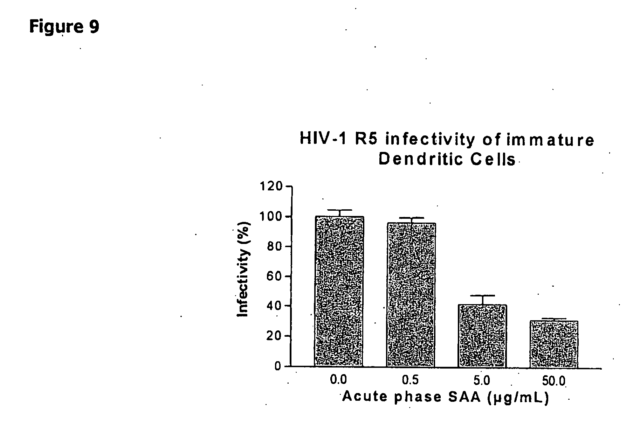

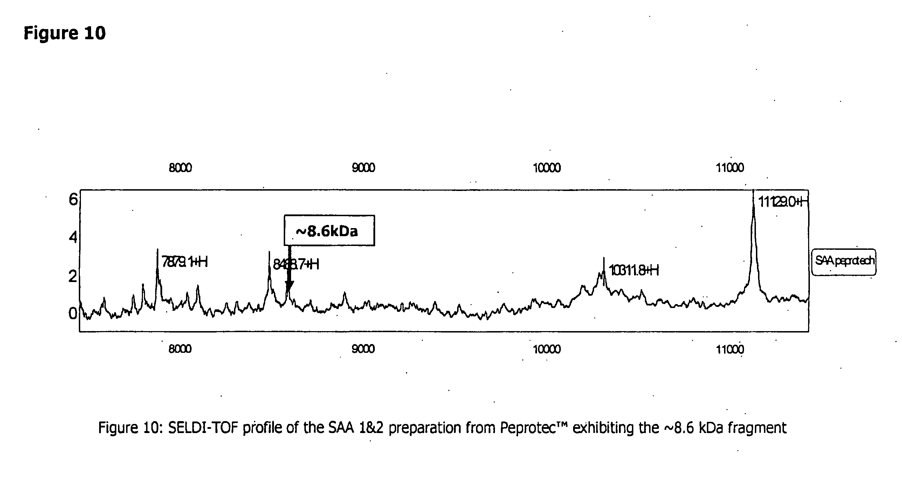

Other characteristics and advantages of the invention will be given in the following examples and with reference to figures 1 to 10, which represent, respectively:
■ Figure 1 : Different steps of the viral envelop attachment to cell receptors,
■ Figure 2: SELDI-TOF protein profile from individuals from different cohorts,
■ Figure 3: Inhibitory effects on HIV-I infection of the cascade infection EU,
■ Figure 4: Depletion of the protein of about 8.6 kDa using an anti-A-SAA Mab,
■ Figures 5 and 6: Comparative IL-8 (Figure 5) and IL-22 (Figure 6) RT-PCR from individuals from different cohorts,
■ Figure 7: Western validate of SAGE analysis from individuals from different cohorts,
■ Figure 8: Induction of VIH-1 CCR5 co-receptor phosphorylation by the binding acute phase SAA protein to the FPR receptor,
■ Figure 9: HIV-1 R5 infectivity of immature dendritic cells,
■ Figure 10: SELDI-TOF profile of SAA preparation exhibiting the ≈ 8.6 kDa fragment.

### Materials and Methods

### Exposed uninfected (EU) individuals recruitment

HIV exposed but uninfected individuals were enrolled in the study. In each case the ESN was the sexual partner of a HIV infected patient; in each couple a prolonged history of penetrative sexual intercourse without condom (and no other known risk factors) was reported. Inclusion criteria for the EU was a history of multiple unprotected sexual episodes for at least four years with at least four episodes of at-risk intercourse within 4 months prior to the study period. EUs were repeatedly HIV seronegative by culture and RNA virus load methods. HIV-infected individuals and healthy controls were also enrolled in the study. HIV patients and HC were age-and-sex-matched with the EU. All EU, HIV+ and HC individuals had been longitudinally followed for at least 4 years (prior to the study period) by the Department of Infectious Diseases, Santa Maria Annunziata Hospital in Florence. This allowed us to exclude from the study ESN and HC in whom sexually transmitted diseases or any other pathology had been reported in that time period. The EU were characterized on the basis of the presence of CCR5-Δ32 alleles; a heterozygous deletion was detected in 1 individual. All EU, HIV patients and low-risk uninfected individuals agreed to donate peripheral blood mononuclear cells.

### Cells

Proteomic and Transcriptomic comparative studies were carried out on T cells from EU and HIV+ forming discordant couples having frequent unprotected sexual intercourse or invasive drug injection by syringe exchanges. T cells from HC were the controls of these analyses. Peripheral blood mononuclear cells (PBMC), obtained from the 3 cohorts: HC, EU and HIV+ were collected and separated over Ficoll-Hypaque, were short-term (6 days) cultivated (Yssel, H. and Spits, H, in Current Protocols in Immunology, Chapter 7.19) then T lymphocytes (CD4+ and CD8+) were CD3/CD28 activated and cultivated in RPMI supplemented of 10% of FCS. Briefly, to activate the CD3-TCR complex, 10 µg/mL of anti-CD3, SPV-T3b monoclonal antibody (MAb) was used to coat 24-well plates for 4 hr at 37°C. Subsequently, 106 cells were then deposited in these coated wells in the presence of culture medium (Yssel's medium, Irvine scientific, Santa Ana, CA) containing 1% of AB+ human serum and 1 µg/mL of anti-CD28 L293 MAb. Three T cells activation times were respectively done 2, 6 and 18 hr. Activated cells pooled from 5 individuals per cohort (having each an equivalent number of cells and total RNA, **Table 2**) for T cell gene expression studies that were carried out using the Serial Analysis Gene Expression (SAGE, Velculescu 1995). Subsequently, a set of total ARN of each individual of the pool was freeze for further use to validate individually the SAGE results. A set of these cells was also used to perform Power and Western blotting analyses (see below). Soluble proteins presents in the plasma of individuals (n=25, **Table 2**) from 3 cohorts were analysed by SELDI-TOF Ciphergen™ approach.

Dendritic cell were derived of monocytes from healthy donors. Briefly, a buffy coat was processed to obtain highly purified monocytes that were cultivated in DMEM medium supplemented of 10% of FCS in the presence of 10 ng/mL of IL-4 and 150 ng of GM-CSF (Becton and Dickinson) for 7 days up to obtain well characterized using appropriated MAbs (anti-DC sign, Anti-CD1a, anti-CD83 and anti-CD86 MAbs) also exhibiting the presence of the Formyl peptide receptor-like 1 (FPRL1) (a receptor belonging to the Formyl Peptide receptor (FPR) family) immature Dendritic Cells (iDC). Cells were maintained at 37°C in a 5% CO2 humid atmosphere.

### Antibodies and Reagents

Recombinant human IL-22, anti-CCR5 polyclonal Ab, anti-human IL-22 polyclonal were purchased from R & D Systems (Oxon, UK), Serum amyloid A (A-SAA) and IL-8 proteins were purchased from Peprotech (Rocky Hill, NJ), MIP-1β was obtained from (Françoise Baleux (Pasteur Institute, Paris, France), Anti-IL-8 MAb was purchased from Bender, Anti-CXCR4, Anti-SAA1 and 2 MAb (Biosource), Anti-SAA MAb (Calbiochem), Anti-active Stat-1 polyclonal Ab, anti- Stat1 MAb, Anti-active Stat-3 polyclonal Ab, Anti-Stat-3 pAb, anti-active Stat-5 polyclonal Ab, Anti-Stat-5 MAb was purchased from Becton and Dickinson (Palo Alto, CA). The anti-SOCS 3 polyclonal Ab (Santa Cruz laboratories, Santa Cruz, CA)

### Plasma analysis by Protein-Chip SELDI-TOF approach

Before analyze, plasma samples were centrifuged at 13 000 rpm during 15 min, the pellet was discarded and supernatant was diluted (1:10) in optimized binding buffer (BB: NaCl 0.250 M Hepes 50 mM, pH 7.5). Diluted plasma samples were applied during 1 hr onto previous saturated strong anion exchanger (SAX2) Protein-chips™ by two BB baths of 5 min. Unbound proteins were washed out using successively 3 washes of 5 min with the washing buffer (WB: NaCl 1M, Hepes 50 mM, pH 7.5) and a final wash using 5 µL of pure bi-distilled water. The Chip-captured proteins were subsequently air-dried at room temperature (RT) before their covering with a matrix (3,5-dimethoxy-4-hydroxycinnapynic acide (SPA) in 99.9% acetonitril and 0.1% trifluoroacetic acid) to absorb the laser energy. The matrix-prepared samples were dried at RT. These samples then received an average of 100 real time laser shots to desorb the captured proteins at a laser intensity that varied from 10 000 up to 40 000 shots (arbitrary units) to generate a protein spectrum (proteogram). The ionized and desorbed proteins were detected and their molecular masses pointed on the proteogram pics were determined using TOF analysis with the Protein-Chip Biology System II software (PBS II; Ciphergen) and the Ciphergen Peaks software. The mass to charge ratio (m/z) of each captured protein by the chip-surface was determined according to externally calibrated standards: human Angiotensin I (1.2965 kilodaltons, kDa), human ACTH (2.9335 kDa), human â-endorphin (3.4650 kDa), bovine insulin (5.7336 kDa), and bovine ubiquitin (8.5648 kDa).

### Depletion of the protein of ∼ 8.6 kDa from EU plasma

Twenty five microliters of magnetic beads (Dynal) washed 3 times with I mL of PBS were added of 25 µg of anti-A-SAA (SAA-1 & SAA-2) MAb from Clinisciences concentrated at 100 µg/mL and incubated for 18 hr at 4°C in orbital shaking. Anti-A-SAA MAb coated beads were subsequently washed 3 times with 1 mL of PBS. Five hundred microliters of EU plasma was then added and incubated at 37°C during 3 hr under shaking. This plasma supernatant was then reanalysed using the appropriated Ciphergen Chip. Five microliters of EU preincubated with anti-A-SAA 1 & 2 MAb or not were applied and analysed as previously indicated by SELDI-Tof (Ciphergen™).

### Inhibition of HIV-1 infecion by recombinant A-SAA protein

Before HIV-1 infection iDC cells were incubated for 1 hr at the designated concentrations with the acute phase human apolipoprotein serum amyloid A (SSA from Peprotec™) which is an agonist of FPRL1. Subsequently, the cells were infected with HIV-1 ADA or HXB2 at an MO1 of 0. 1 for 2 hours. The cells were extensively washed and incubated in complete medium. HIV-1 p24 levels were determined by enzyme-linked immunosorbent assays (Beckman-Coulter, France) 4 days after infection.

### SAGE Analysis

SAGE was performed essentially as outlined in the detailed of Velculescu's protocol obtainable at the URL:WWW.sagenet.org with the modifications of Powell and Kenzelmann.

### Power Blot Analysis

Immunoblot analysis of proteins was carried out as described (www.translab.com/shtml). Briefly, CD3/CD28-stimulated T cells from the 3 cohorts were lysed by the lysis buffer (Tris 10 mM pH 7.4, Na+ orthovanadate I mM, SDS 1%), sonicated and clarified by centrifugation. Proteins were migrated in 5-15% gradient SDS-polyacrylamide gels to detect a wide size range of proteins in one gel. Four hundred micrograms of protein was loaded in long well across the entire width of the gel. This translates into near 15 µg of protein electrophoresed per lane on a standard 25-well gel. Subsequently the gel was transferred to Immobilon-P membrane (Millipore, Bedford, MA) overnight. After transfer, membranes were blocked for 1hr with 5% milk. Subsequently, the membrane was inserted into a Western blotting manifold that isolates 45 channels across the membrane. In each channel, different complex antibody cocktails were added and allowed to hybridize for 1 hr. Following staining, the membranes were washed and hybridised for 30 min with secondary goat anti-mouse horseradish peroxidase (HRP). All antibodies were mouse monoclonal. Membranes were washed and developed with SuperSignal West Pico (Pierce, Santa Clara, CA).

### RT-PCR analysis

Total RNA, isolated from activated T cells was converted by reverse transcription into cDNA. For each total RNA sample reverse transcription at 42°C for 50 min, the following reagents were used: 1 µg total RNA and 200 Units Superscript II reverse transcriptase (RT, Gibco-BRL); RT buffer as supplied; 100 mmol/L dithiothreitol (DTT), 40 units of Rnasin (Promega, Madison, WI, USA); 1.25 mmol/L of each dNTP; and 500 ng of oligo dTs. PCR was performed as follow: 2 µL cDNA; 1.25 mmo/L of each dNTP, 2.5 units Taq polymerase (Promega); 2.5 mmol/L MgCl2, 2.5 µL 10X buffer and 20 pmol of each specific primer pair in a 25 µL total volume. The following specific primers were used: IL-22: SEQ ID N°1 sense 5'-TGACAAGTCCAACTTCCAGCAG-3', SEQ ID N°2 antisense 5'-TCTGGATATGCAGGTCATCACC-3'; IL-8: SEQ ID N°3 sense 5'-AACTTCTCCACAACCCTCTG-3', SEQ ID N°4 antisense 5'-TTGGCAGCCTTCCTGATT-3'; GAPDH: SEQ ID N°5 sense 5'- CCA-CCC-ATG-GCA-AAT-TCC-ATGGCA-3' and SEQ ID N°6 antisense 5'-TCTAGACGGCAGGTCAGGTCCACC-3'. After preincubation (94°C, 5 min), each PCR sample underwent a 29 cycles amplification regimen of denaturation (94°C, I min), primer annealing (56°C, I min) and primer extension (72°C, 1 min) with a final extension (72°C, 10 min).

### Western blotting analysis

One million of CD3/CD28 activated T cells (as indicated above) from each cohort (HC, EU, and HIV+) were lysed in a 1% NP40 buffer. For each group, equal amounts of protein were electrophoresed under reducing conditions and transferred electrophoretically to nitrocellulose membranes. Membranes were incubated for 30 min in TBS (50 mmol/L NaCI, 20 mmol/L Tris HCl, pH 7.5) containing 5% BSA and 0.1% Tween 20 and then incubated overnight at 4 °C with a primary antibody. Proteins were visualized using the ECL system (Amersham Pharmacia Biotech, Piscataway, NJ). Blots were washed in TBS containing 0.1 % Tween 20 and incubated with HRPconjugated goat anti-rabbit or anti-mouse secondary antibody (Amersham Pharmacia Biotech, Piscataway, NJ). For reblotting with another antibody, filters were stripped as previously described [10].

### HIV-1 coreceptor phosphorylation assessing

Immature Dendritic cells were stimulated with MIP-1β or with the acute phase A-SAA (Peprotec™) at the indicated (in Fig 7) concentrations for the indicated periods of time at 37°C. Then the cells were lysed after 20 min on ice with periodic mixing in lysis buffer (1% Triton X-100, 20 mM Tris HCl pH 8.0, 137 mM NaCl, 15% glycerol, 5 mM EDTA) containing phosphatase inhibitors (1 mM phenylsulfonyl fluoride, 5 µg/mL aprotinin, 5 µg/mL leupeptin, 1 mM sodium orthovanadate, 1 mM EGTA). Cell lysates were precleaned with 30 µL of washed protein A Sepharose beads (15 µL packed beads) at 4°C for 1 hr and 1 µg of polyclonal anti-phosphoserine antibody (BD) was added to 200 µg cell lysates. The reaction mixture was incubated at 4°C overnight. The immune complex was captured by adding 50 µL of washed protein A sepharose beads (25 µL packed beads). The reaction mixture was incubated at 4°C for an additional 2 hours. The beads were spun down (10 sec at 14000 rpm), drained off the supernatant, washed 3 times with ice cold I X IP buffer, then were resuspended in 30 µL 2 X Laemli sample buffer and boiled for 5 min to eluate the immune complex. After electrophoresis on 10% SDS-PAGE precast gel (Invitrogen), the proteins were transferred to nitrocellulose membranes. CCR5 was visualized using a polyclonal anti-CCR5 (R & D Systems) and ECL system (Amersham Pharmacia Biotech, Piscataway, NJ).

### Human chemokine, Searchlight™, arrays

Four different plasma from each studied cohort were analysed following the instructions the manufacturer of chemokine Searchlight arrays (Pierce Endogen, Perbio, Boston) for the plasma content in 8 chemokines.

### Results

Despite being repeatedly exposed to Human Immunodeficiency Type I virus (HIV-1) via sexual or systemic routes certain individuals remain uninfected. To investigate the molecular mechanisms underlying resistance to HIV-1 infection, the inventors have performed a comparative study on CD3/CD28-activated peripheral blood T cells (to enhance cell signalling and gene expression) and plasma (to study their soluble proteins) from cohorts of HIV-1 exposed uninfected individuals (EU), their HIV-1-infected sexual partners and healthy controls (**Table 2**).

| **n.** | **category** | **HIV+ partner of each EU** | **viral load** | **CD4** |
|---|---|---|---|---|
| | | | | |
| EU | | HIV+ | | |
| 1 | EU1 | HIV+ 20 | 460 | 348 |
| 2 | EU2 | HIV+ 21 | < 400 | 244 |
| 3 | EU3 | HIV+ 22 | 400 | 327 |
| 4 | EU4 | HIV+ 23 | 9420 | 328 |
| 5 | EU5 | HIV+ 24 | 9440 | 916 |
| 6 | EU6 | HIV+ 18 | <50 | 205 |
| 7 | EU7 | HIV+ 25 | 750000 | 16 |
| 8 | EU8 | HIV+ 26 | 2070 | 636 |
| 9 | EU9 | HIV+ 27 | 399 | 101 |
| 10 | EU10 | HIV+ 28 | 750000 | 424 |
| 11 | EU11 | HIV+ 9 | <50 | 673 |
| 12 | EU12 | HIV+ 29 | <50 | |
| 13 | EU13 | HIV+ 16 | <50 | 472 |
| 14 | EU14 | HIV+ 19 | <50 | 1220 |
| 15 | EU15 | HIV+ 13 | <50 | 321 |
| 16 | EU16 | HIV+ 30 | <400 | 385 |
| 17 | EU17 | HIV+ 31 | >750000 | 49 |
| 18 | EU18 | HIV+ 32 | 400 | 339 |
| 19 | EU19 | HIV+ 33 | <50 | |
| 20 | EU20 | HIV+ 34 | 400 | 327 |
| 21 | EU21 | HIV+ 35 | 350000 | 166 |
| | | | | |
| HC | | | | |
| 1 | HC1 | | HIVneg | |
| 2 | HC2 | | HIVneg | |
| 3 | HC3 | | HIVneg | |
| 4 | HC4 | | HIVneg | |
| 5 | HC5 | | HIVneg | |
| 6 | HC6 | | HIVneg | |
| 7 | HC7 | | HIVneg | |
| 8 | HC8 | | HIVneg | |
| 9 | HC9 | | HIVneg | |
| 10 | HC10 | | HIVneg | |
| 11 | HC11 | | HIVneg | |
| 12 | HC12 | | HIVneg | |
| 13 | HC13 | | HIVneg | |
| 14 | HC14 | | HIVneg | |
| 15 | HC15 | | HIVneg | |
| 16 | HC16 | | HIVneg | |
| 17 | HC17 | | HIVneg | |
| 18 | HC18 | | HIVneg | |
| 19 | HC19 | | HIVneg | |
| 20 | HC20 | | HIVneg | |
| 21 | HC21 | | HIVneg | |
| 22 | HC22 | | HIVneg | |

Complementary gnomic, proteomic and cell signalling analyses were carried out using Serial Analysis Gene Expression (SAGE), Surface-Enhanced Laser Desorption lonisation and Time Of Fly Mass Spectrophotometry (SELDI-TOF, Ciphergen™) and Power blotting™, respectively (see Material and 6 Method Section). Understanding of the genetic and physiology of the Long term non progressors (LTNP) and EU individuals with respect to natural anti-viral mechanisms could provide the basis of the treatment against HIV infection. The inventors have then studied physiopathological mechanisms on the basis of the absence of infection in individuals subject to frequent exposures to HIV in EU individuals.

First results obtained from the high number of gene tags (HC: 21193 tags, EU: 22697 tags, and HIV+: 17 285 tags) of transcriptome analyses by the SAGE method exhibited that in EU were found to overexpress the **Th1 IL-22** and **SOCS1** and that **Granzyme B** was to underexpress in HIV+ compared to EU and HC cohorts that exhibited similar levels **(Table 3)** these results of course were obtained without having any "a priori" idea.

### SAGE

### Gene level expression

| GENE | HC | ESN | HIV + |
|---|---|---|---|
| IL - 22 | 1 | 13 | 0 |
| SOCS 1 | 0 | 3 | 0 |
| Granzyme B | 3.91 | 3.96 | 0 |

### Power blot

### Protein level expression

| PROTEIN | HC | ESN | HIV + |
|---|---|---|---|
| STAT3 | 0 | 5 | 0 |

In parallel using Power Blot analysis of proteins from pooled T cells from the 3 cohorts **the acute-phase response factor STAT3** was detected. The plasma analyses (using SELDI-TOF approach) from 25 individuals per cohort have shown an expression increase of a soluble protein of a MW of ∼8.6 kDa, (**Fig. 2**).

Taking into account that **IL-22** initiates a cascade (**Fig. 3**) of innate immune response that includes the **Jack/STAT** pathway, **SOCS 3, beta-defensins,** and the acute phase apolipoprotein serum amyloid A **(A-SAA) :** SAA-1 and SAA2 , these data were further developed using different methods to confirm and extend their signification (see Material and Method Section). Synthesised in the liver and in other tissues as epitheliums of blood vessels, the ASAA is found associated to HDL and HDL-free in the plasma. The A-SAA promoter is highly responsive to inflammatory cytokines such as IL1β, TNFα; IFNγ and IL6 that can be induced by LPS. Moreover recently, it has been shown that the Th1 IL-22 cytokine is able to participate to A-SAA expression. These observations have suggested that A-SAA could play a role as an immune innate defense molecule at local sites. Post transductional cleavage of A-SAA produces C-term fragments of approximately 8.5 kDa MW. To identify the ∼8.6 kDa protein obtained from the SELDI-TOF analysis, a specific anti-A-SAA MAb before the plasma SELDI-TOF profiling was used and as shown in **Fig. 4** the pic corresponding to ∼8.6 kDa was depleted using this anti-A-SAA MAb. Interestingly, it has been also known that A-SAA is able to induce the IL-8 cytokine then an IL-8 specific RT-PCR was performed from 5 individual of each cohort to verify that this cytokine characterize the EU cascade. **Figure 5** clearly shows that the PCR evidence a specific polymorphism of IL-8 in EU in comparison with the 2 other groups (HIV+ and HC).A specific IL-22 RT -PCR was also performed (**Fig. 6**) in each individual that formed the pools and we could observe that only EU overexpress significantly this important Th1 cytokine.

Other controls and validations were done, thus Western Blot analyses were done on proteins from pooled samples. It was also observed in EU group that STAT1 and STAT3 were phosphorylated (**Fig. 7A** and **7B**), whereas STAT5 was down regulated in HIV+ but higher and equal level of activation in both HC and EU groups (**Fig. 7C**). The expression of SOCS3 protein (**Fig. 7D**), a STAT3 responsive gene, was upregulated in EU. Furthermore, these EU individuals were shown over-express alpha-defensins. Moreover, it has been shown that IL-8 is able to desensitize HIV coreceptors through FPR receptors family and that Stat1 is necessary for cell antiviral factor (CAF)-mediated inhibition of HIV-1 Long terminal repeat (LTR) activation and HIV replication.

Since agonists of FPR and FPRL1, soluble A-SAA, the WKYMWVm peptide, the bacterial chemotactic peptide fMLF, and probably its ∼8.6 kDa fragment), induce phosphorylation as shown in our experiments using A-SAA in specific Western Blotings (**Fig. 8**) and downmodulation of both the HIV-1 coreceptor CCR5 and CXCR4 through FPR activation . Then as reported [31,38], it has been shown that A-SAA protein inhibited HIV-I infection (**Fig. 9**).
The A-SAA purchased from Peprotec™ used in these assays exhibit the ∼8.6 kDa fragment (**Fig. 10**)

These results allowed to identify a cascade of events that favour the innate host resistance to HIV infection characterizing EU. Since IL-22 is able via JAK/STAT to induce the Beta defensins, A-SAA and that A-SAA induces IL8 secretion of its expression, these results clearly depicted this cascade. Moreover, IL-8 and β-Defensin have been shown to decrease HIV-I infection. Altogether our results show that IL-22 is a starter cytokine helping the innate immune response that provides resistant mechanisms to HIV infection (**Fig.2**).

Additionally the SAGE analysis interestingly shows that Granzyme B was down regulated in HIV+ but maintained in EU and HC individuals, This confirm the observation of the loss of granzyme made in HIV-HAART treated individuals [2, 3]. The inventors have also observed in SAGE analysis that a higher production of **IFN-gamma** in EU than in HC and HIV+. These cytokine is typically antiviral which has been found in some studies on EU made by others.

Taking into account that the cascade of events was found to induce several and major elements of the innate immunity, the scope of the invention also extends to other viruses and retroviruses than HIV.

It will also be considered that the EU exhibited higher amounts of phosphorylated STAT1 and that importantly this element is essential to the activity of the "cell anti-viral Factors" (CAF) secreted by CD8 T cells. It has also been shown that HIV+ appears to loose the Granzymes B in comparison with EU and HC. Granzymes B is produced by CD8 T cells and NK to kill infected cells. The STAT-1 dependent production of CAF Granzymes B plays major role in the anti-HIV activity in persons that resist to AIDS development despite their HIV infection.

It has also been observed in SAGE analysis that a higher production of IFN-gamma in EU than in HC and HIV+. These cytokine is typically antiviral.

These cascades elements should be involved not only as element of the resistance to the viral infection but also as element of the resistance to the induced disease.

## Claims

1. IL-22 for use in preventing infections diseases by initiating innate immune response.

2. IL-22 for use according to claim 1, wherein IL-22 is in a form of cytokine in association with a pharmaceutically inert vehicle.

3. IL-22 for use according to claim 1 or 2, wherein the drugs are under formulations for oral or mucosal applications or by injection.

4. IL-22 for use according to claim 3, wherein, for oral application, the drugs are presented in the form of tablets, pills, capsules, drops, patch or spray.

5. IL-22 for use according to claim 4, wherein for application by injection, the drugs are under the form of solution for injection by the intravenous, subcutaneous, or intramuscular route produced from sterile or sterilisable solution, or suspension or emulsion.

6. IL-22 for use according to claim 4, wherein, for mucosal application, the drugs are under the form of gels.

## Patentansprüche

1. IL-22 zur Verwendung zur Prävention von Infektionskrankheiten durch Auslösen einer angeborenen Immunantwort.

2. IL-22 zur Verwendung gemäß Anspruch 1, wobei IL-22 in Form des Cytokins in Verbindung mit einem pharmazeutisch inerten Träger vorliegt.

3. IL-22 zur Verwendung gemäß Anspruch 1 oder 2, wobei die Wirkstoffe als Zubereitungen für die orale oder mukosale Anwendung oder für die Anwendung durch Injektion vorliegen.

4. IL-22 zur Verwendung gemäß Anspruch 3, wobei die Wirkstoffe für die orale Anwendung in Form von Tabletten, Pillen, Kapseln, Tropfen, Pflaster oder Spray vorliegen.

5. IL-22 zur Verwendung gemäß Anspruch 4, wobei die Wirkstoffe für die Anwendung durch Injektion in Form einer Lösung für die Injektion auf intravenösem, subkutanem oder intramuskulärem Weg vorliegen, die aus einer sterilen oder sterilisierbaren Lösung oder Suspension oder Emulsion hergestellt ist.

6. IL-22 zur Verwendung gemäß Anspruch 4, wobei die Wirkstoffe für die mukosale Anwendung in Form von Gelen vorliegen.

## Revendications

1. IL-22 pour utilisation dans la prévention de maladies infectieuses en initiant une réponse immunitaire innée.

2. IL-22 pour utilisation selon la revendication 1, **caractérisé en ce que** IL-22 est sous forme de cytokine, en association avec un véhicule pharmaceutiquement inerte.

3. IL-22 pour utilisation selon la revendication 1 ou 2, **caractérisé en ce que** les médicaments sont sous des formulations pour des applications orale ou muqueuse ou par injection.

4. IL-22 pour utilisation selon la revendication 3, **caractérisé en ce que**, pour une application orale, les médicaments sont présentés sous forme de comprimés, pilules, capsules, gouttes, patch ou spray.

5. IL-22 pour utilisation selon la revendication 4, **caractérisé en ce que** pour une application par injection, les médicaments sont sous forme de solution pour injection par voie intraveineuse, sous-cutanée ou intramusculaire, produite à partir de solution stérile ou stérilisable, ou de suspension ou d'émulsion.

6. IL-22 pour utilisation selon la revendication 4, **caractérisé en ce que, pour une** application par voie muqueuse, les médicaments sont sous forme de gels.
